# EUROPÄISCHE PATENTSCHRIFT

(11) **EP 1 283 730 B1**
(45) Veröffentlichungstag und Bekanntmachung des Hinweises auf die Patenterteilung: **14.07.2004**
(21) Anmeldenummer: 01944998.2
(22) Anmeldetag: 09.04.2001
(51) Int. Cl.: A61L 27/36

(54) **KIEFERKNOCHEN-TRANSPLANTAT AUS NATÜRLICHEM KNOCHENMATERIAL**
JAW TRANSPLANT CONSISTING OF NATURAL BONE MATERIAL
IMPLANT DE L'OS MAXILLAIRE EN MATERIAU OSSEUX NATUREL

(30) Priorität: 26.05.2000 DE 10026306
(43) Veröffentlichungstag der Anmeldung: 19.02.2003
(73) Patentinhaber: Tutogen Medical GmbH, 91077 Neunkirchen am Brand (DE)
(72) Erfinder: HEERKLOTZ, Klaus, 90513 Zirndorf (DE); KOSCHATZKY, Karl, 91058 Erlangen (DE); KRÜGER, Manfred, 61389 Arnoldshain (DE); FÖHLINGER, Bernd, 91054 Erlangen (DE)
(74) Vertreter: Manitz, Finsterwald & Partner GbR
(86) Internationale Anmeldenummer: PCT/EP2001/004053
(87) Internationale Veröffentlichungsnummer: WO 2001/091818

(56) Entgegenhaltungen:
- DE-A- 2 754 917
- DE-A- 4 226 465
- US-A- 5 556 430
- KABAN L.B. ET AL.: "Treatment of Jaw Defects with Demineralized Bone Implants" J. ORAL MAXILLOFAC. SURG., Bd. 40, 1. Januar 1982 (1982-01-01), Seiten 623-626, XP001025749

## Beschreibung

Die vorliegende Erfindung betrifft ein Transplantat zur anatomischen Wiederherstellung der Knochenform eines defekten oder atrophischen Kieferkammes (Onlay-Sandwich-Augmentation).

Die Erfolgssicherheit enossaler Transplantationsverfahren wurde ausreichend wissenschaftlich bewiesen. Die Implantologie gilt heute bei einem ausreichendem ortsständigen Knochenangebot, bei strenger Indikationsstellung, sorgfältiger operativer Technik und exakter prothetischer Versorgung als eine klinisch etablierte Behandlungsmaßnahme.

Aus der US-A-5,556,430 ist eine flexible Matrix aus demineralisiertem Knochenmateriäl bekannt, die eine ausreichende Flexibilität aufweist, um ein Anformen an den umgebenden Bereich des Skelettes zu ermöglichen. Die Membran kann als "Poncho" für einen Wundverschluss verwendet werden.

Die Aufgabe der vorliegenden Erfindung besteht darin, ein verbessertes Kieferknochentransplantat zu schaffen, das den Heilungsprozess beim Patienten beschleunigt.

Die Lösung dieser Aufgabe erfolgt durch die Merkmale des Anspruchs 1.

Das erfindungsgemäße Transplantat ist aufgrund seiner rinnenförmig gewölbten Ausbildung einerseits an die anatomische Form des Kieferknochens angepasst. Andererseits kann hierdurch in den Defekt zur Unterfütterung eingebrachtes Knochenersatzmaterial (Knochenpulver oder Knochenchips) ortsständig gehalten und darüberhinaus die Augmentation fehlenden Knochens bewirkt werden.

Das Transplantat dient ferner als stabile Abdeckung der mit Knochenmahlgut aufgefüllten Defektstelle und verhindert damit eine Migration des Knochenersatzmaterials. Das erfindungsgemäße Transplantat schädigt das Transplantatlager nicht und beeinflußt bildgebende Verfahren nicht bzw. nur geringfügig- Die anatomische Form eines defekten oder atrophischen Kieferkammes und/ oder seiner Seitenwände kann durch das erfindungsgemäße Transplantat wiederhergestellt werden.

Ein besonderer Vorteil des Transplantates gemäß der Erfindung ist durch das verwendete Material gegeben, das aufgrund seines biologischen Ursprungs keinen Fremdkörper darstellt. Dadurch trägt das aus Knochenmaterial hergestellte Transplantat zur Fixation und Fusion zwischen Transplantat und Transplantatlager bei, indem es sich während der Einheilung in körpereigenes Gewebe umwandelt.

Das erfindungsgemäße Transplantat kann sowohl im gesamten Ober- als auch im Unterkiefer für augmentative Maßnahmen in den verschiedenen Ausformungen verwendet werden sowie für eine horizontale als auch vertikale Knochengewinnung (Kieferkammverbreiterung und Kieferkammerhöhung), für die spätere oder gleichzeitige Implantation einer metallischen Prothese, und für die Wiederherstellung von Knochendefekten allgemein.

Durch die rinnenförmig gewölbte Ausbildung des Transplantatkörpers ist gewährleistet, daß einerseits eine Anpassung an die Anatomie des Kieferknochens erfolgen kann. Andererseits kann das Kieferknochentransplantat dazu verwendet werden, in die Defektstelle eingebrachtes Knochenersatzmaterial ortsständig zu halten. Nach einer weiteren Ausbildung der Erfindung ist -hierzu der Transplantatkörper im Querschnitt im wesentlichen U-förmig ausgebildet, so daß dieser zwei parallele oder annähernd parallel verlaufende Seitenwände aufweist, die eine Verlängerung zur wangenseitigen Kieferwand bzw. zur zungenseitigen Kieferwand bilden, wodurch der Kieferkamm mit Hilfe des Transplantats tunnelformig überbaut werden kann.

In der Beschreibung, der Zeichnung und den Unceransprüchen sind weitere vorteilhafte Ausführungsformen des Kieferkriochentransplantates gemäß der vorliegenden Erfindung angegeben.

Nach einer ersten vorteilhaften Ausführungsform, der Erfindung kann der Transplantatkörper eine oder mehrere Öffnungen, Durchgangsbohrungen oder Schlitze aufweisen. Derartige Öffnungen können einerseits zur Anbringung einer Fixierung durch Stifte, Schrauben oder Nägel dienen. Weiterhin beschleunigen derartige Öffnungen auch den biologischen Umbau und sie können Verwendung zur späteren Einführung eines metallischen Implantates zur prothetischen Versorgung finden.

Die Geometrie des rinnenförmig gewölbt ausgebildeten Transplantatkörpers kann je nach Anforderung unterschiedlich sein. Die Oberflächen des Transplantatkörpers können - im mathematischen Sinn - stetig oder unstetig verlaufen, d.h. der durch den Transplantatkörper gebildete Tunnebogen kann gekrümmt, kontinuierlich gewölbt, jedoch auch aus unstetig aneinandergrenzenden Teilstücken gebildet sein, so daß die Außen- und/oder Innenkontur des Transplantatkörpers im Querschnitt gesehen einen Polygonzug bildet. Die beiden Schenkel eines im wesentlichen U-förmig ausgebildeten Transplantatkörpers können durch einen Verbindungsabschnitt einstückig miteinander verbunden sein, der - im Querschnitt gesehen - eben ausgebildet ist oder als Teilkreis (z.B. Viertelkreis oder Halbkreis) ausgebildet ist, wodurch der Winkel, den die Schenkel des Transplantatkörpers miteinander einschließen, vorgegeben wird. Im Falle eines viertelkreisförmigen Verbindungsabschnittes ergibt sich demnach nach wie vor ein rinnenförmig gewölbt ausgebildeter Transplantatkörper, der jedoch im Querschnitt gesehen eher V-förmig ausgebildet ist.

Das erfindungsgemäße Kieferknochentransplantat kann wie oben beschrieben Wandabschnitte aufweisen, die parallel zu einander verlaufen oder in einem Winkel zueinander stehen. Diese Wandabschnitte können die gleiche Höhe besitzen oder unterschiedlich hoch sein, so daß sich ein etwa L-förmiger Querschnitt ergibt.

Nach einer bevorzugten Ausführungsform der Erfindung besteht das Material des Transplantatkörpers aus konserviertem und sterilem Knochenmaterial humanen oder tierischen Ursprungs, insbesondere aus konserviertem und sterilem Knochenmaterial bovinen, porcinen oder equinen Ursprungs.

Das Material kann erfindungsgemäß aus prozessiertem, konserviertem und sterilem Knochenmaterial humanen Ursprungs, sogenanntem Allograft, oder aus prozessiertem, konserviertem und sterilem Knochenmaterial tierischen Ursprungs, sogenanntem Xenograft bestehen. Ferner kann das Knochenmaterial aus spongiösem, kortikalem oder kompaktem Knochen bzw. aus daraus resultierenden Verbunden bestehen und gegebenenfalls mit Knochenwachstumsfaktoren (BMP's) beladen sein.

Gemäß der vorliegenden Erfindung kann ein geeignetes allogenes oder xenogenes Knochenmaterial derart prozessiert werden, daß es konserviert, lagerfähig sowie steril ist und bestimmungsgemäß eingesetzt werden kann. Die Konservierung des Knochenmaterials kann beispielsweise mittels Gefriertrocknung erfolgen. Vorzugsweise wird aber das Knochenmaterial durch Lösungsmittel-Dehydratisierung von kollagenem Knochenmaterial mittels eines organischen mit Wasser mischbaren Lösungsmittels, z.B. Methanol, Ethanol, Propanol, Isopropanol; Aceton, Methyl-Ethylketon oder Gemischen dieser Lösungsmittel, erzeugt. Die Konservierung und Sterilisation des Knochenmaterials nach diesem Verfahren ist auch Gegenstand des Patents DE 29 06 650.

Dieses Verfahren dient der Herstellung von Transplantatkonserven und- ermöglicht eine Dehydratisierung und Freilegung bis in den Feinbau des Materials, so daß das prozessierte Knochenmaterial im histologischen Bild eine dem natürlichen Knochen sehr ähnliche Scruktur aufweist und somit die gewünschten Eigenschaften des Ausgangsmaterials erhalten bleiben. Dieses Verfahren der Lösungsmittel-Dehydratisierung hat außerdem den Vorteil, daß im Vergleich zur Gefriertrocknung ein wesentlich geringerer apparativer Aufwand erforderlich ist.

Ferner kann das Knochenmaterial auch durch Lösungsmitteldehydratisierung von kollagenhaltigem Knochenmaterial mit anschließender terminaler Sterilisation, insbesondere durch Bestrahlung mit Gamma- bzw. Elektronenstrahlen, aber auch durch Ethylenoxid oder thermischer Verfahren hergestellt werden.

Alternativ kann das Knochenmaterial durch aseptische Prozessierung von kollagenhaltigem Knochenmaterial ohne terminale Sterilisation erzeugt werden, wobei auch eine vollständige oder teilweise Demineralisierung möglich ist. Die Demineralisierung des Knochenmaterials nach diesem Verfahren ist auch Gegenstand der deutschen Patentanmeldung 19849984.1.

Eine weitere Möglichkeit besteht darin, das konservierte Knochenmaterial mit Knochenwachstumsfaktoren (BMP's) zu beladen, um den Heilungsprozeß zu beschleunigen.

Das Transplantat kann in seiner Größe an den abzudeckenden Defekt angepaßt werden, was durch Zurichten von standardisierten Ausformungen des Transplantates erfolgt.

Im Bereich der frontalen Kieferzone kann ein Transplantatkörper mit einer Breite von 8mm ausreichend sein. Ansonsten sind die Außenabmessungen je nach Einsatzort verschieden und betragen beispielsweise: Länge (L) etwa 8 bis 22 mm, Breite (B) etwa 7 bis 8 mm, Wandstärke (W) etwa 1 bis 2 mm, Höhe (H) etwa 7 bis 10 mm.

Zur Stabilisierung und Adaption kann das aus Knochenmaterial geformte Transplantat mittels Schrauben, Stiften oder Nägeln im vorhandenen Eigenknochen fixiert werden("Onlay-Sandwich-Augmentation"). Anschließend ist über das Transplantat eine Membran zu legen, da ein dichter Wundverschluß eine wichtige Voraussetzung für eine komplikationslose Einheilung des Augmentates ist.

Der Zeitverlauf der Einheilung ist abhängig von der Reagibilität des Lagers und dauert Wochen bis Monate. In der Regel ist die Einheilung nach 6-8 Monaten soweit fortgeschritten, daß beispielsweise in das neu aufgebaute Knochenlager eine Transplantatinsertion durchgeführt werden kann. Eine Kontrolle der Einheilung kann mittels via Röntgen, Biopsie, CT oder anderer diagnostischer Methoden erfolgen.

Nachfolgend wird die vorliegende Erfindung rein exemplarisch anhand von Ausführungsbeispielen und unter Bezugnahme auf die beigefügten Zeichnungen beschrieben. Es zeigen:
- Fig. 1: eine perspektivische Ansicht sowie eine Querschnittsansicht einer ersten Ausführungsform eines Kieferknochentransplantats;
- Fig. 2: eine perspektivische Ansicht sowie eine Querschnittsansicht einer zweiten Ausführungsform eines Kieferknochentransplantats;
- Fig. 3: eine perspektivische Ansicht sowie eine Querschnittsansicht einer dritten Ausführungsform eines Kieferknochenimplantats;
- Fig. 4: eine Querschnittsansicht eines atrophischen Kieferkamms mit augmentativer Maßnahme;
- Fig. 5: eine Querschnittsansicht eines durch Augumentation erhöhten Kieferkammes;
- Fig. 6: eine Querschnittsansicht eines atrophischen Kiefers mit augmentativer Maßnahme; und
- Fig. 7: eine Querschnittsansicht eines Kiefers mit augmentativer Maßnahme.

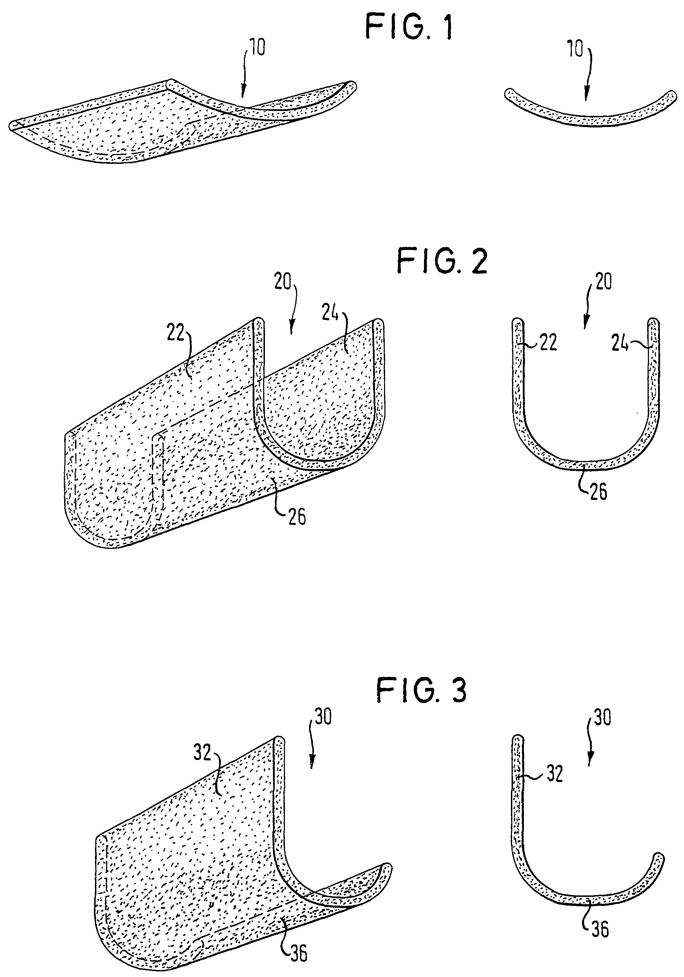

Das in Fig. 1 dargestellte Kieferknochenimplantat besteht aus einem rinnenförmig gewölbt ausgebildeten Transplantatkörper 10, der einstückig aus spongiösem, kortikalem oder kompaktem Knochenmaterial menschlichen oder tierischen Ursprungs hergestellt ist. Der Transplantatkörper bildet eine in Längsrichtung geradlinig und quer zur Längsrichtung rinnenförmig gekrümmt ausgebildete Schale. Der Transplantatkörper 10 ist in Draufsicht rechteckig ausgebildet.

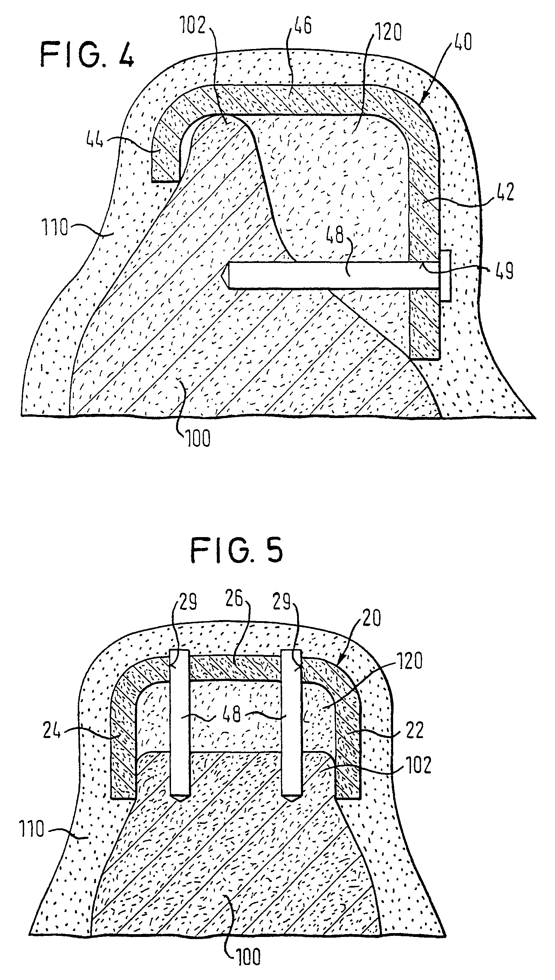

Fig. 2 zeigt eine weitere Ausführungsform eines Transplantatkörpers 20, der aus dem gleichen Knochenmaterial wie der Transplantatkörper 10 hergestellt ist und der ebenfalls rinnenförmig gewölbt ausgebildet ist. In der Querschnittsansicht von Fig. 2 ist zu erkennen, daß der Transplantatkörper 20 im Querschnitt die Form eines U aufweist, wobei die Schenkel des U Seitenwände 22, 24 bilden, die im wesentlichen parallel zueinander verlaufen und die durch einen gewölbten Rinnenabschnitt 26 miteinander verbunden sind. Bei diesem Ausführungsbeispiel besitzen die beiden Wandabschnitte 22 und 24 die gleiche Höhenerstreckung.

Fig. 3 zeigt eine dritte Ausführungsform eines Kieferknochentransplantates, das aus einem rinnenförmig gewölbt ausgebildeten Transplantatkörper 30 besteht, der im wesentlichen der Ausführungsform von Fig. 2 entspricht, wobei jedoch der in Fig. 2 dargestellte rechte Wandabschnitt 24 weggenommen ist. Insofern besteht der Transplantatkörper 30 lediglich aus einem Wandabschnitt 32, der einstückig mit einem gekrümmt ausgebildeten Rinnenabschnitt 36 verbunden ist.

In Fig. 4 ist eine Querschnittsansicht eines atrophischen Kieferkammes 102 dargestellt, an dem eine augmentative Maßnahme vorgenommen worden ist. Hierzu ist ein erfindungsgemäßes Kieferknochentransplantat 40 vorgesehen, das im wesentlichen dem Ausführungsbeispiel von Fig. 3 entspricht, wobei jedoch neben einem Wandabschnitt 42 und einem Rinnenabschnitt 46 ein weiterer, kurzer Wandabschnitt 44 vorgesehen ist, der etwa parallel zum Wandabschnitt 42 verläuft, jedoch nur etwa 30 % seiner Höhenerstreckung aufweist. Wie bei diesem Ausführungsbeispiel zu erkennen ist, kann der Boden bzw. das Dach des Rinnenabschnittes 46 eben ausgebildet sein, d.h. eine durchgehend gekrümmte Ausbildung ist nicht erforderlich.

Bei der Anordnung von Fig. 4 ist das Kieferknochentransplantat 40 zusätzlich mit Hilfe von Stiften 48 in dem Kieferknochen 100 fixiert, wobei die Stifte 48 durch Bohrungen 49 in dem Transplantatkörper 46 gesteckt sind. Eine Fixierung der Kieferknochentransplantate kann mit Hilfe von Stiften, Nägeln, Schauben oder dergleichen erfolgen. Eine zusätzliche Fixierung mit einer Membran über dem Transplantat kann erfolgen, um eine Immigration des Weichteilgewebes zu verhindern.

Wie Fig. 4 zeigt, ist derjenige Hohlraum, der durch den Kieferknochen 100 bzw. den Kieferkamm 102 und den Transplantatkörper 40 gebildet ist, mit Knochenersatzmaterial 120 in Form von Knochenpulver oder Knochenspänen ausgefüllt.

Fig. 5 zeigt eine Anwendung des Kieferknochentransplantates 20 von Fig. 2 zur Erhöhung des Kieferkamms durch Augmentation. Hierbei ist das im Querschnitt U-förmige Kieferknochentransplantat 20 auf einen Kieferkamm 102 eines Kieferknochens 100 derart aufgesetzt, daß die Wandabschnitte 22 und 24 die wangenseitige Kieferwand sowie die zungenseitige Kieferwand verlängern, wobei zwischen dem Transplantatkörper 20 und dem Kieferkamm 102 ein Hohlraum gebildet ist, der mit Knochenersatzmaterial 120 ausgefüllt ist. Auch bei diesem Ausführungsbeispiel ist zusätzlich zur Fixierung des Transplantatkörpers 20 mit Hilfe der Schleimhaut 110 eine Befestigung mit Hilfe von Stiften 48 vorgesehen, die durch Bohrungen 29 in dem Rinnenabschnitt 26 des Transplantatkörpers 20 gesteckt und in dem Kieferknochen 100 fixiert sind.

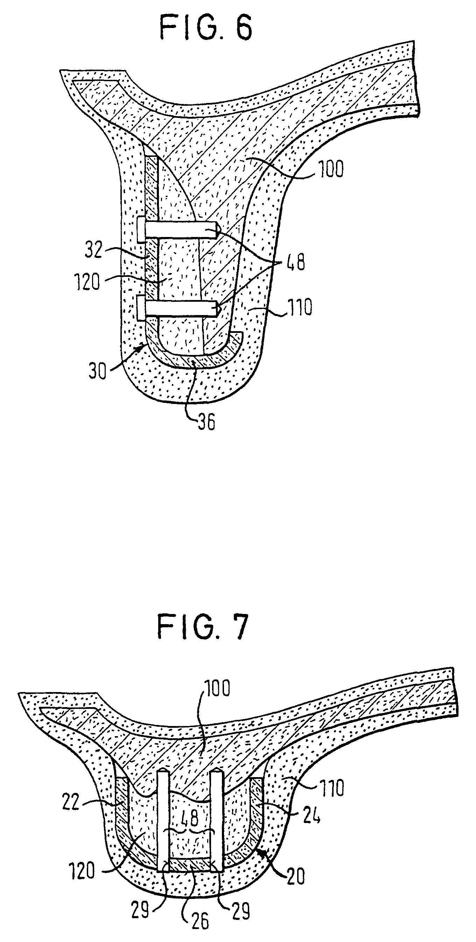

Fig. 6 zeigt eine Anwendung des Kieferknochentransplantates 30 von Fig. 3 bei der Vergrößerung eines atrophischen Kiefers. Wie hier zu erkennen ist, bildet der Wandabschnitt 32 des Kieferknochentransplantates 30 eine wangenseitige Kieferwand nach, wobei der Rinnenabschnitt 36 das untere Ende des noch vorhandenen Kieferknochens 100 teilweise umgreift. Im übrigen bezeichnen in dieser Figur wie auch in den übrigen Figuren gleiche Bezugszeichen gleiche Teile, so daß zur Vermeidung von Wiederholungen auf die Beschreibung der vorherigen Figuren verwiesen werden kann.

In Fig. 7 ist die Augmentation eines Kiefers mit Hilfe des Kieferknochentransplantates 20 dargestellt, das - ähnlich wie bei Fig. 5 - mit Hilfe von Stiften 48 im Kieferknochen 100 fixiert ist. Auch hier ist eine zusätzliche Fixierung einer Membran über dem Transplantat möglich, um eine Immigration des Weichteilgewebes zu verhindern.

Mit Hilfe des erfindungsgemäßen Kieferknochentransplantates kann durch Ansetzen des Transplantatkörpers an den Kieferknochen ein Hohlraum geschaffen werden, in den Knochenersatzmaterialien eingebracht und ortsfest gehalten werden können. Die hierfür erforderliche rinnenförmige Wölbung kann durch gekrümmte oder ebene Teilabschnitte des Transplantatkörpers realisiert werden.

### Bezugszeichenliste

- 10, 20, 30: Transplantatkörper
- 22, 24: Wandabschnitt
- 26: rinnenförmiger Abschnitt
- 29: Bohrungen
- 32: Wandabschnitt
- 36: rinnenförmiger Abschnitt
- 40: Transplantatkörper
- 42: Wandabschnitt
- 44: Wandabschnitt
- 46: rinnenförmiger Abschnitt
- 48: Stift
- 49: Bohrung
- 100: Kieferknochen
- 102: Kieferkamm
- 110: Schleimhaut
- 120: Knochenersatzmaterial

## Patentansprüche

1. Kieferknochentransplantat, bestehend aus einem Transplantatkörper (10, 20, 30, 40) aus spongiösem, kortikalem oder kompaktem Knochenmaterial menschlichen oder tierischen Ursprungs, der eine rinnenförmig gewölbt ausgebildete Schale bildet.

2. Kieferknochentransplantat nach Anspruch 1,
**dadurch gekennzeichnet,**
**daß** der Transplantatkörper (10, 20, 30, 40) eine oder mehrere Öffnungen (29, 49), Durchgangsbohrungen oder Schlitze aufweist.

3. Kieferknochentransplantat nach Anspruch 1,
**dadurch gekennzeichnet,**
**daß** der Transplantatkörper in Längsrichtung gekrümmt ausgebildet ist.

4. Kieferknochentransplantat nach Anspruch 1,
**dadurch gekennzeichnet,**
**daß** der Transplantatkörper (20, 30, 40) im Querschnitt im wesentlichen U-förmig ausgebildet ist.

5. Kieferknochentransplantat nach Anspruch 1,
**dadurch gekennzeichnet,**
**daß** der Transplantatkörper (40) im Querschnitt im wesentlichen U-förmig ausgebildet ist, wobei die Schenkel (42, 44) des U unterschiedlich lang sind.

6. Kieferknochentransplantat nach einem der vorangehenden Ansprüche,
**dadurch gekennzeichnet,**
**daß** das Material des Transplantatkörpers (10, 20, 30, 40) aus konserviertem und sterilem Knochenmaterial besteht.

7. Kieferknochentransplantat nach einem der vorangehenden Ansprüche,
**dadurch gekennzeichnet,**
**daß** das Knochenmaterial durch Lösungsmitteldehydratisierung von kollagenem Knochenmaterial mittels eines organischen, mit Wasser mischbaren Lösungsmittels, z.B. mittels Methanol, Ethanol, Propanol, Isopropanol, Aceton, Methyl-Ethylketon oder Gemischen dieser Lösungsmittel, erzeugt ist.

8. Kieferknochentransplantat nach einem der vorangehenden Ansprüche,
**dadurch gekennzeichnet,**
**daß** das Knochenmaterial durch Lösungsmitteldehydratisierung von kollagenem Knochenmaterial mit anschließender terminaler Sterilisation, insbesondere durch Bestrahlung mit Gamma- oder Elektronenstrahlen erzeugt ist.

9. Kieferknochentransplantat nach einem der vorangehenden Ansprüche 1 - 5,
**dadurch gekennzeichnet,**
**daß** das Knochenmaterial durch aseptische Prozessierung von kollagenem Knochenmaterial, insbesondere mit vollständiger oder teilweiser Demineralisierung, ohne terminale Sterilisation erzeugt ist.

10. Kieferknochentransplantat nach einem der vorangehenden Ansprüche,
**dadurch gekennzeichnet,**
**daß** das Knochenmaterial mit zumindest einem Knochenwachstumsfaktor (BMP) beladen ist.

## Claims

1. A jawbone transplant consisting of a transplant body (10, 20, 30, 40) made of a spongeous, cortical or compact bone material of human origin or of animal origin, which forms a shell made in an arched channel-like manner.

2. A jawbone transplant in accordance with claim 1, **characterized in that** the transplant body ( 10, 20, 30, 40) has one or more openings (29, 49), throughbores or slits.

3. A jawbone transplant in accordance with claim 1, **characterized in that** the transplant body is made curved in the longitudinal direction.

4. A jawbone transplant in accordance with claim 1, **characterized in that** the transplant body (20, 30, 40) is made substantially in a U shape in cross-section.

5. A jawbone transplant in accordance with claim 1, **characterized in that** the transplant body (40) is made substantially in a U shape in cross-section, with the limbs (42, 44) of the U being of different length.

6. A jawbone transplant in accordance with any one of the preceding claims, **characterized in that** the material of the transplant body (10, 20, 30, 40) consists of preserved and sterile bone material.

7. A jawbone transplant in accordance with any one of the preceding claims, **characterized in that** the bone material is produced by solvent dehydration of collagenic bone material by means of an organic solvent miscible with water, e.g. by means of methanol, ethanol, propanol, isopropanol, acetone, methyl ethyl ketone or mixtures of these solvents.

8. A jawbone transplant in accordance with any one of the preceding claims, **characterized in that** the bone material is produced by solvent dehydration of collagenic bone material with subsequent terminal sterilization, in particular by radiation with gamma rays or electron beams.

9. A jawbone transplant in accordance with any one of the preceding claims 1 - 5, **characterized in that** the bone material is produced by aseptic processing of collagenic bone material, in particular with complete or partial demineralization, without terminal sterilization.

10. A jawbone transplant in accordance with any one of the preceding claims, **characterized in that** the bone material is charged with at least one bone growth factor (BMP).

## Revendications

1. Transplant d'os maxillaire, consistant en un corps formant transplant (10, 20, 30, 40) en un tissu osseux spongieux, cortical ou compact d'origine humaine ou animale, qui constitue une coquille réalisée arquée et en forme de gouttière.

2. Transplant d'os maxillaire selon la revendication 1,
**caractérisé en ce que** le corps formant transplant (10, 20, 30, 40) présente une ou plusieurs ouvertures (29, 49), alésages traversants ou fentes.

3. Transplant d'os maxillaire selon la revendication 1,
**caractérisé en ce que** le corps formant transplant est réalisé recourbé dans le sens longitudinal.

4. Transplant d'os maxillaire selon la revendication 1,
**caractérisé en ce que** le corps formant transplant (20, 30, 40) est réalisé avec une section transversale sensiblement en forme de U.

5. Transplant d'os maxillaire selon la revendication 1,
**caractérisé en ce que** le corps formant transplant (40) est réalisé avec une section transversale sensiblement en forme de U, les branches (42, 44) du U étant de longueurs différentes.

6. Transplant d'os maxillaire selon l'une des revendications précédentes,
**caractérisé en ce que** la matière du corps formant transplant (10, 20, 30, 40) consiste en un tissu osseux préservé et stérile.

7. Transplant d'os maxillaire selon l'une des revendications précédentes,
**caractérisé en ce que** le tissu osseux est obtenu par déshydratation par solvant du tissu osseux collagène à l'aide d'un solvant organique, propre à être mélangé avec de l'eau, par exemple à l'aide de méthanol, d'éthanol, de propanol, d'isopropanol, d'acétone, de méthyléthylcétone ou de mélanges de ces solvants.

8. Transplant d'os maxillaire selon l'une des revendications précédentes,
**caractérisé en ce que** le tissu osseux est obtenu par déshydratation par solvant du tissu osseux collagène, avec ensuite une stérilisation finale, notamment par exposition à des rayons gamma ou à des faisceaux électroniques.

9. Transplant d'os maxillaire selon l'une des revendications 1 à 5,
**caractérisé en ce que** le tissu osseux est obtenu par un traitement aseptique de tissu osseux collagène, notamment par déminéralisation totale ou partielle, sans stérilisation finale.

10. Transplant d'os maxillaire selon l'une des revendications précédentes,
**caractérisé en ce que** le tissu osseux comporte au moins un facteur de croissance osseuse (BMP).
